# EUROPEAN PATENT SPECIFICATION

(11) **EP 3 173 105 B1**
(45) Date of publication and mention of the grant of the patent: **08.09.2021**
(21) Application number: 16190790.2
(22) Date of filing: 27.09.2016
(51) Int. Cl.: A61L 29/02, A61L 29/08, A61L 29/14, A61L 29/06

(54) **METAL MEDICAL DEVICE AND METHOD FOR PRODUCING THE SAME**
METALLISCHE MEDIZINISCHE VORRICHTUNG UND VERFAHREN ZUR HERSTELLUNG DAVON
DISPOSITIF MÉDICAL MÉTALLIQUE ET SON PROCÉDÉ DE FABRICATION

(30) Priority: 26.11.2015 JP 2015230841
(43) Date of publication of application: 31.05.2017
(73) Proprietor: Sumitomo Rubber Industries, Ltd., Kobe-shi, Hyogo-ken 651-0072 (JP)
(72) Inventor: MINAGAWA, Yasuhisa, Kobe-shi Hyogo 651-0072 (JP); NAKASHITA, Takefumi, Kobe-shi Hyogo 651-0072 (JP)
(74) Representative: Manitz Finsterwald Patent- und Rechtsanwaltspartnerschaft mbB

(56) References cited:
- WO-A1-2015/060129
- US-A1- 2003 215 649
- US-A1- 2011 086 234
- US-A1- 2011 159 101
- Gelest Inc.: "Hydrophobicity, Hydrophilicity and Silane Surface Modification", , 24 May 2011 (2011-05-24), pages 1-82, XP055098863, Retrieved from the Internet: URL:https://acs.expoplanner.com/files/acsf all13/ExhibFiles/1047_1100_HydrophobicityC atalogUS_5-24-11.pdf [retrieved on 2014-01-28]

## Description

### TECHNICAL FIELD

The present invention relates to a method for producing a metal medical device.

### BACKGROUND ART

Guide wires, stents, or other medical devices which are inserted into blood vessels, respiratory tracts, urethras, or other body cavities or tissues and then optionally placed there may damage the body tissues or the like and cause inflammation or pain to the patient upon insertion. Accordingly, it is desired to improve the sliding properties of the medical devices upon insertion into the body. Moreover, if proteins or cells are adsorbed onto the surface of stents or other medical devices intended to be placed in the body for a long period of time, they can cause problems such as formation of blood clots clogging blood vessels. Accordingly, there is another need to reduce such adsorption as much as possible.

Methods to solve these problems have been proposed including, for example, a method for improving the sliding properties of medical devices such as guide wires or stents by coating the surface of the medical devices with a hydrophilic resin, a fluororesin, or the like to impart lubricity to the surface.

However, such methods including coating the surface with resin and methods further including curing after coating can show problems, especially for metal medical devices. For example, the resin coat may be easily peeled off or detached from the surface, resulting in reduced sliding properties, or proteins or cells may gradually adhere to the surface of the medical devices indwelling in the body. Therefore, a need exists for metal medical devices having good ability to reduce deterioration of sliding properties, good low protein or cell adsorption properties.
US 2011 / 0086 234 A1 discloses a NO-releasing sol-gel-coating formed from a sol precursor solution comprising a backbone alkoxysilane and a diazeniumdiolate-modified alkoxysilane. WO 2015/060129 A1 and EP 3 061 470 A1 disclose a metal medical device and a method for producing the metal device comprising treating a surface of a metal medical device with a silane compound containing a functional group that is a polyoxyalkylene group, a metal salt-containing hydrophilic group, a halogen salt-containing hydrophilic group, or a fluorine-containing hydrophobic group.
US 2011 / 159 101 A1 discloses an implantable or insertable medical device comprising a polymeric matrix comprising a hydrophilic polymer having pendent groups comprising a reacted silyl ether group and a method for providing a coating to a medical device.

### SUMMARY OF INVENTION

### TECHNICAL PROBLEM

The present invention aims to solve the above problem and provides a method for producing a silane-functionalized metal medical device having better durability in time, i.e. is excellent in the ability to reduce deterioration of sliding properties, low protein adsorption properties, and low cell adsorption properties.

### SOLUTION TO PROBLEM

The invention is defined by the claims. A silane-functionalized metal medical device is produced, having a surface treatment layer on at least a part of its metal surface, the surface treatment layer including: a primer layer formed of an alkoxysilane represented by the formula (I) : R¹¹ₖSi (OR¹²)₄₋ₖ (I), wherein each R¹¹ or each R¹² is the same or different and represents a C1-C8 saturated or unsaturated hydrocarbon group, and R¹¹ and R¹² are the same as or different from each other, and k represents an integer of 0 to 3; and a functional layer on the primer layer, the functional layer being formed of a silane compound containing a polyoxyalkylene group, a metal salt-containing hydrophilic group, a halogen salt-containing hydrophilic group, or a fluorine-containing hydrophobic group.

The metal salt-containing hydrophilic group is preferably an alkali metal salt-containing hydrophilic group or an alkaline earth metal salt-containing hydrophilic group.

The halogen salt-containing hydrophilic group is preferably a chlorine salt-containing hydrophilic group.

The fluorine-containing hydrophobic group is preferably a perfluorooxyalkylene group.

The present invention relates to a method for producing the above-described silane-functionalized metal medical device, the method including: step 1 of priming a metal surface with an alkoxysilane; and step 2 of functionalizing the primed surface using a silane compound containing a polyoxyalkylene group, a metal salt-containing hydrophilic group, a halogen salt-containing hydrophilic group, or a fluorine-containing hydrophobic group. The method further includes step 3 of maintaining a functionalized product obtained in step 2 at a humidity of 50% or more. The alkoxysilane is represented by the formula (I): R¹¹ₖSi(OR¹²)₄₋ₖ (I), wherein each R¹¹ or each R¹² is the same or different and represents a C1-C8 saturated or unsaturated hydrocarbon group, and R¹¹ and R¹² are the same as or different from each other, and k represents an integer of 0 to 3.

Step 2 is preferably carried out at a pH of 5 or lower.

### ADVANTAGEOUS EFFECTS OF INVENTION

The silane-functionalized metal medical device of the present invention has a surface treatment layer on at least a part of its metal surface, the surface treatment layer including: a primer layer formed of an alkoxysilane represented by the formula (I); and a functional layer on the primer layer, the functional layer being formed of a silane compound containing a polyoxyalkylene group, a metal salt-containing hydrophilic group, a halogen salt-containing hydrophilic group, or a fluorine-containing hydrophobic group. Such a metal medical device has better durability in time, i.e. is excellent in the ability to reduce deterioration of sliding properties, low protein adsorption properties, and low cell adsorption properties.

### DESCRIPTION OF EMBODIMENTS

The metal medical device of the present invention has a surface treatment layer on at least a part of its metal surface. The surface treatment layer includes a primer layer formed of an alkoxysilane represented by the formula (I), and a functional layer on the primer layer. The functional layer is formed of a silane compound containing a polyoxyalkylene group, a metal salt-containing hydrophilic group, a halogen salt-containing hydrophilic group, or a fluorine-containing hydrophobic group.

Metal medical devices produced by conventional surface treatment methods, such as, for example, by coating the surface with resin, have a problem in maintaining sliding properties (i.e. the durability of sliding properties) as the resin coating layer (lubrication layer) may be easily peeled or detached by stress, such as rubbing with a hand, due to the absence of a chemical bond between the metal surface and the resin coating layer, and other reasons. Furthermore, another problem is that the lubrication layer may flow away during the long-term indwelling period, resulting in deterioration of low protein adsorption properties and low cell adsorption properties.

In contrast, since the metal medical device of the present invention has on its metal surface a primer layer formed of a primer selected from an alkoxysilane represented by the formula (I), and further has on the primer layer a functional layer formed of a silane compound containing a polyoxyalkylene group, a metal salt-containing hydrophilic group, a halogen salt-containing hydrophilic group, or a fluorine-containing hydrophobic group, the silane compound is firmly fixed to the metal surface via the primer. Accordingly, not only is a functional layer (lubrication layer) that is excellent in sliding properties, low protein adsorption properties, and low cell adsorption properties formed, but peeling or detachment of the functional layer by stress or other factors, exfoliation of the functional layer during the long-term indwelling period, and other problems are reduced so that the above properties can be prevented from deteriorating, thereby resulting in excellent durability.

Moreover, when the silane compound containing a polyoxyalkylene group, a metal salt-containing hydrophilic group, or a halogen salt-containing hydrophilic group is used, a larger amount of water molecules will be held around these functional groups due to their very high affinity for water, and such water molecules provide fluid lubrication. Accordingly, the metal medical device functionalized using such a silane compound has significantly improved sliding properties. In addition, the lubrication layer is sufficiently prevented from flowing away during the long-term indwelling period, and thus deterioration of low protein adsorption properties and low cell adsorption properties can also be prevented.

When the silane compound containing a fluorine-containing hydrophobic group is used, on the other hand, the functional group significantly reduces the surface tension, thereby greatly decreasing the adhesive force of the molecules between the surface of the metal medical device and an object contacting the surface (for example, the inner surface of a catheter or body cells when the metal medical device is a guide wire). This greatly improves the sliding properties of the metal medical device. Also in the case of such a silane compound, the lubrication layer is sufficiently prevented from flowing away during the long-term indwelling period, and thus deterioration of low protein adsorption properties and low cell adsorption properties can be prevented as well.

The metal medical device of the present invention is produced by a method including step 1 of priming a metal surface with an alkoxysilane represented by formula (I), and step 2 of functionalizing the primed surface using a silane compound containing a polyoxyalkylene group, a metal salt-containing hydrophilic group, a halogen salt-containing hydrophilic group, or a fluorine-containing hydrophobic group, wherein the method comprises step 3 of maintaining a functionalized product obtained in step 2 at a humidity of 50% or more.

In step 1, a metal surface is primed with an alkoxysilane represented by the formula (I). The metal surface of an untreated metal medical device is treated with at least one primer selected from the alkoxysilane represented by the formula (I) to cover the metal surface with a primer layer formed of the primer.

The priming treatment in step 1 may be carried out by any method that allows the primer to contact the metal surface of an untreated metal medical device. Examples include a method of applying or spraying a liquid primer or a primer solution to the metal surface, and a method of immersing an untreated metal medical device in a liquid primer or a primer solution. The priming treatment may be appropriately followed by washing, drying, or other operations in accordance with known methods. The primer solution may be prepared using a solvent, concentration, and other conditions selected appropriately according to the type of primer.

Before step 1, the untreated metal medical device may be subjected to washing with acetone, ethanol, or other solvents, drying and other operations, if necessary. The conditions for washing or drying may be chosen appropriately.

The alkoxysilane represented by the formula (I) as the primer may be selected appropriately in view of adhesion to the silane compound containing a polyoxyalkylene group or the like which covers the primer layer. The alkoxysilane preferably has 1 to 22 carbon atoms, more preferably 1 to 16 carbon atoms because then the effects of the present invention can be well achieved. Although any compound containing a known alkoxy group may be used as the alkoxysilane, from the same standpoint as above, it preferably contains at least one selected from the group consisting of a methoxy group, an ethoxy group, a propoxy group, and a butoxy group. It more preferably contains an ethoxy group and/or a butoxy group, among these, and still more preferably contains an ethoxy group and a butoxy group.

Examples of the alkoxysilane include monoalkoxysilanes having one alkoxy group, dialkoxysilanes having two alkoxy groups, trialkoxysilanes having three alkoxy groups, and tetraalkoxysilanes having four alkoxy groups, which are compounds represented by the following formula (I):

R¹¹ₖSi(OR¹²)₄₋ₖ (I)

wherein each R¹¹ or each R¹² is the same or different and represents a C1-C8 saturated or unsaturated hydrocarbon group, and R¹¹ and R¹² are the same as or different from each other;
and k represents an integer of 0 to 3.

Among these, compounds having two or more alkoxy groups are preferred, and compounds having three or more alkoxy groups are more preferred.

R¹¹ and R¹² each preferably have 1 to 6 carbon atoms, more preferably 1 to 4 carbon atoms. R¹¹ and R¹² are each preferably a saturated hydrocarbon group.

Specific examples of the monoalkoxysilanes include trimethylmethoxysilane, trimethylethoxysilane, trimethylpropoxysilane, triethylmethoxysilane, triethylethoxysilane, and tripropylpropoxysilane. Specific examples of the dialkoxysilanes include dimethyldimethoxysilane, dimethyldiethoxysilane, dimethyldipropoxysilane, diethyldimethoxysilane, diethyldiethoxysilane, methylethyldimethoxysilane, methylethyldiethoxysilane, and dipropyldimethoxysilane. Specific examples of the trialkoxysilanes include methyltrimethoxysilane, methyltriethoxysilane, methyltripropoxysilane, ethyltrimethoxysilane, ethyltriethoxysilane, propyltrimethoxysilane, propyltriethoxysilane, and butyltrimethoxysilane. Specific examples of the tetraalkoxysilanes include tetramethoxysilane, tetraethoxysilane, tetrapropoxysilane, tetrabutoxysilane, dibutoxydiethoxysilane, and butoxytriethoxysilane.

The alkoxysilane in the present invention may also suitably be a compound represented by the following formula (II) :

(R²¹O)ₘSi(OR²²)ₙ (II)

wherein each of R²¹ and R²² is the same or different and represents a C1-C8 saturated or unsaturated hydrocarbon group; and m+n = 4 with m≥n≥0 on average.

R²¹ and R²² each preferably have 1 to 6 carbon atoms, more preferably 1 to 4 carbon atoms. R²¹ and R²² are each preferably a saturated hydrocarbon group.

Examples of the silane compound represented by formula (II) include a butoxy/ethoxy type tetraalkoxysilane ("Primer coat PC-3B" available from Fluoro Technology) represented by the following formula (II-1):

(H₃C-CH₂-CH₂-CH₂-O-)ₘ-Si-(O-CH₂-CH₃)ₙ

wherein m+n = 4 with m>n>0 on average.

In step 2, the surface primed in step 1 (the surface of the primer layer formed on the metal medical device) is further functionalized using a silane compound containing a polyoxyalkylene group, a metal salt-containing hydrophilic group, a halogen salt-containing hydrophilic group, or a fluorine-containing hydrophobic group. By further treatment of the primer layer with the certain silane compound, the primer layer is covered with a functional layer formed of the silane compound. This allows the silane compound to be firmly fixed to the metal surface via the primer, as a result of which various functions can be imparted including sliding properties, low protein adsorption properties, low cell adsorption properties, and the durability of these properties.

The functionalization in step 2 may be carried out by any method that allows the certain silane compound to contact the surface of the primer layer formed on the metal medical device. Examples include a method of applying or spraying a liquid silane compound or a silane compound solution to the surface of the primer layer, and a method of immersing the metal medical device with a primer layer formed thereon in a liquid silane compound or a silane compound solution. The functionalization may be appropriately followed by washing, drying, and other operations in accordance with known methods. The silane compound solution may be prepared using a solvent, concentration, and other conditions selected appropriately according to the type of silane compound.

In order to promote the hydrolysis reaction, the functionalization in step 2 is preferably carried out using a liquid silane compound or silane compound solution adjusted to have a pH of 5 or lower, more preferably 2 to 4. The pH may be adjusted by conventionally known methods, such as addition of an acid or alkali. Examples of the acid include inorganic acids such as sulfuric acid, nitric acid, and hydrochloric acid, and organic acids such as acetic acid. Examples of the alkali include ammonia water, sodium hydroxide, and potassium hydroxide.

The silane compound containing a polyoxyalkylene group, a metal salt-containing hydrophilic group, a halogen salt-containing hydrophilic group, or a fluorine-containing hydrophobic group to be used in step 2 may be any silane compound having at least one group selected from these functional groups .

The silane compound containing a polyoxyalkylene group may be, for example, a silane compound containing a hydrolyzable group and a polyoxyalkylene group in a molecule. Examples of the hydrolyzable group include a methoxy group and an ethoxy group. Examples of the polyoxyalkylene group include divalent groups having a linear or branched polyalkylene ether structure such as a polyoxymethylene group and a polyoxyethylene group.

The silane compound containing a polyoxyalkylene group may suitably be, for example, a silane compound represented by the formula (1) or (2) below. 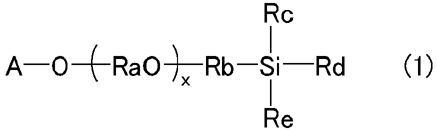 In formula (1), A represents a hydrogen atom, a linear or branched C1-C3 alkyl group, or a linear or branched C1-C3 acyl group; Ra represents a linear or branched C1-C3 alkylene group; Rb represents a linear or branched C1-C5 alkylene group; Rc, Rd, and Re are the same as or different from each other and each represent a halogen atom, a hydroxy group, a linear or branched C1-C3 alkyl group, or a linear or branched C1-C3 alkoxy group, provided that at least one of Rc, Rd, and Re is a halogen atom, a hydroxy group, or a linear or branched C1-C3 alkoxy group; and x represents the number of oxyalkylene repeating units represented by RaO and is an integer of 2 to 500.

From the standpoint of affinity for water, A is preferably a hydrogen atom, a linear or branched C1-C2 alkyl group, or a linear or branched C1-C2 acyl group. From the same standpoint, Ra is preferably a methylene group, an ethylene group, a propylene group, or an isopropylene group, while Rb is preferably a linear or branched C1-C3 alkylene group.

From the standpoint of affinity for water, Rc, Rd, and Re are each preferably a halogen atom, a linear or branched C1-C3 alkyl group, or a linear or branched C1-C3 alkoxy group. In particular, at least one of Rc, Rd, and Re is more preferably a halogen atom such as a chlorine atom or a linear C1-C3 alkoxy group, still more preferably a chlorine atom or a linear C1-C2 alkoxy group. From the same standpoint as above, x is preferably 4 to 200, more preferably 5 to 200. 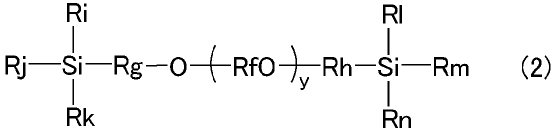 In formula (2), Rf represents a linear or branched C1-C3 alkylene group; Rg and Rh are the same as or different from each other and each represent a linear or branched C1-C5 alkylene group; Ri, Rj, Rk, Rl, Rm, and Rn are the same as or different from each other and each represent a halogen atom, a hydroxy group, a linear or branched C1-C3 alkyl group, or a linear or branched C1-C3 alkoxy group, provided that at least one of Ri, Rj, Rk, Rl, Rm, and Rn is a halogen atom, a hydroxy group, or a linear or branched C1-C3 alkoxy group; and y represents the number of oxyalkylene repeating units represented by RfO and is an integer of 2 to 500.

From the standpoint of molecular rotational mobility or flexibility, Rf is preferably a linear or branched C2-C3 alkylene group. From the standpoint of hydrophilicity or hydrolyzability, Rg and Rh are each preferably a linear or branched C1-C3 alkylene group.

From the standpoint of hydrophilicity or hydrolyzability, Ri, Rj, Rk, Rl, Rm, and Rn are each preferably a linear or branched C1-C3 alkyl group or a linear or branched C1-C3 alkoxy group. In particular, at least one of Ri, Rj, Rk, Rl, Rm, and Rn is more preferably a linear C1-C2 alkoxy group. From the standpoint of molecular rotational mobility or flexibility, y is preferably 4 to 200, more preferably 5 to 200.

Suitable examples of the compound represented by formula (1) include compounds represented by the formulas (1-1) to (1-3) below. Suitable examples of the compound represented by formula (2) include compounds represented by the formulas (2-1) and (2-2) below.

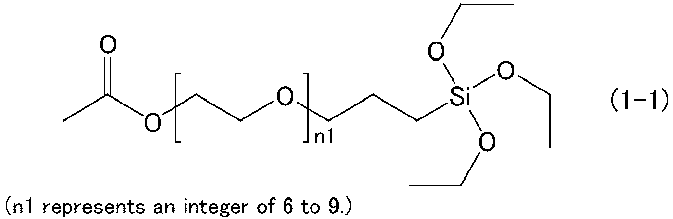

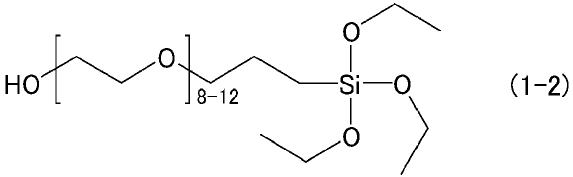

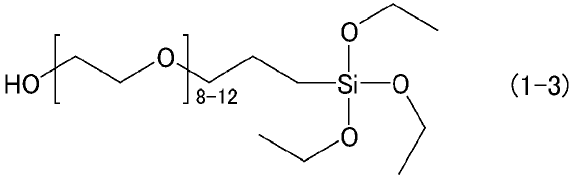

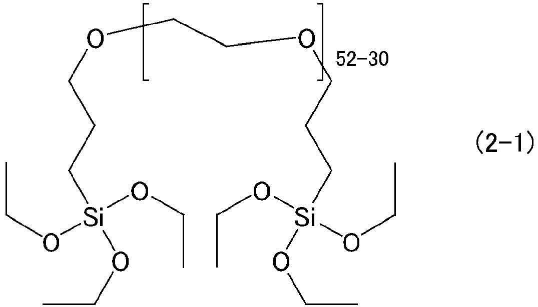

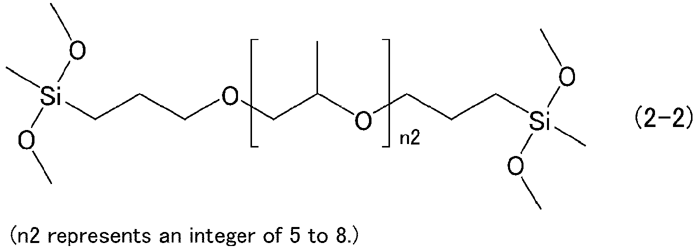

Examples of commercial products of the silane compound containing a polyoxyalkylene group include SIA0078.0 (a compound represented by formula (1-1)), SIH6188.0 (a compound represented by formula (1-2)), SIM6492.57 (a compound represented by formula (1-3)), SIB1824.84 (a compound represented by formula (2-1)), and SIB1660.0 (a compound represented by formula (2-2)), all available from Gelest.

The silane compound containing a metal salt-containing hydrophilic group may be, for example, a silane compound containing a hydrolyzable group and a metal salt-containing hydrophilic group in a molecule. Examples of the hydrolyzable group include a methoxy group and an ethoxy group. Examples of the metal salt-containing hydrophilic group include metal carboxylates, metal phosphonates, and metal sulfonates. The metal of the metal salt-containing hydrophilic group is not particularly limited. For improved water-holding properties, the metal is preferably an alkali metal such as lithium, sodium, or potassium, or an alkaline earth metal such as magnesium or calcium.

The silane compound containing a metal salt-containing hydrophilic group is preferably a compound containing an alkoxysilyl group that contains a hydrolyzable group, such as a methoxysilyl group (e.g. a trimethoxysilyl group) or an ethoxysilyl group (e.g. a triethoxysilyl group), or a compound containing a hydroxysilyl group (e.g. a trihydroxysilyl group), and particularly a compound containing an alkoxysilyl group.

In particular, the silane compound containing a metal salt-containing hydrophilic group may suitably be, for example, a silane compound represented by the formula (3) or (4) below. 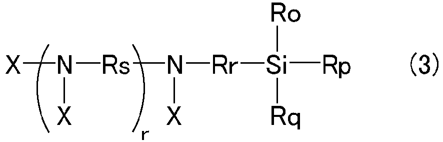 In formula (3), Ro, Rp, and Rq are the same as or different from each other and each represent a halogen atom, a hydroxy group, a linear or branched C1-C3 alkyl group, or a linear or branched C1-C3 alkoxy group, provided that at least one of Ro, Rp, and Rq is a halogen atom, a hydroxy group, or a linear or branched C1-C3 alkoxy group; Rr and Rs are the same as or different from each other and each represent a linear or branched C1-C7 alkylene group; each X is the same or different and represents a metal salt-containing hydrophilic group; and r represents 0 or 1.

From the standpoint of affinity for water, at least one of Ro, Rp, and Rq is preferably a linear C1-C3 alkoxy group or a hydroxy group. From the same standpoint, Rr and Rs are each preferably a linear or branched C1-C5 alkylene group, more preferably a linear or branched C1-C4 alkylene group, still more preferably a linear or branched C1-C3 alkylene group.

Examples of X (metal salt-containing hydrophilic group) include three types of functional groups represented by the formulas below. From the standpoints of lubrication properties and sliding properties, X is preferably a functional group (metal sulfonate-containing group) represented by the formula at the bottom below, among the three. 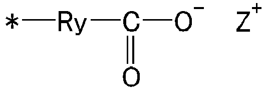 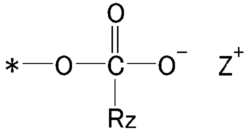 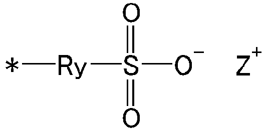 In the formulas, Ry represents a linear or branched C1-C5 alkylene group; Rz represents a linear or branched C1-C3 alkyl group; Z⁺ represents a monovalent metal ion; and * represents a bond. 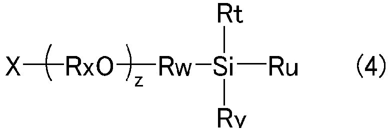 In formula (4), Rt, Ru, and Rv are the same as or different from each other and each represent a halogen atom, a hydroxy group, a linear or branched C1-C3 alkyl group, or a linear or branched C1-C3 alkoxy group, provided that at least one of Rt, Ru, and Rv is a halogen atom, a hydroxy group, or a linear or branched C1-C3 alkoxy group; Rw represents a linear or branched C1-C5 alkylene group; Rx represents a linear or branched C1-C3 alkylene group; X represents a metal salt-containing hydrophilic group; and z represents the number of oxyalkylene repeating units represented by RxO and is an integer of 0 to 500.

From the standpoint of affinity for water, at least one of Rt, Ru, and Rv is preferably a linear C1-C3 alkoxy group or a hydroxy group. From the same standpoint, Rw is preferably a linear or branched C1-C4 alkylene group, more preferably a linear or branched C1-C3 alkylene group. From the same standpoint, Rx is preferably a C1-C2 alkylene group.

The symbol X (metal salt-containing hydrophilic group) is as described for X in formula (3). From the same standpoint as above, when the oxyalkylene group represented by RxO is present, z is preferably 2 to 500, more preferably 5 to 200, still more preferably 7 to 200.

Suitable examples of the compounds represented by formulas (3) and (4) include compounds represented by the formulas (3-1), (4-1), and (4-2) below.

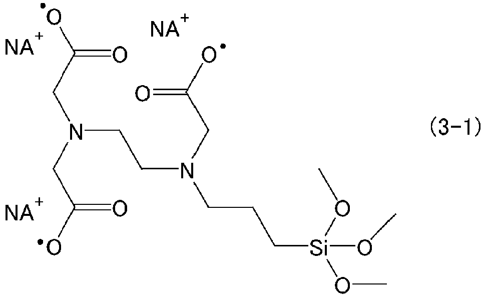

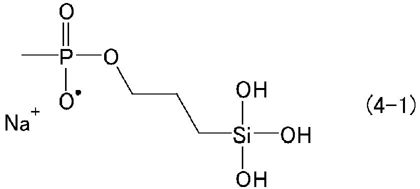

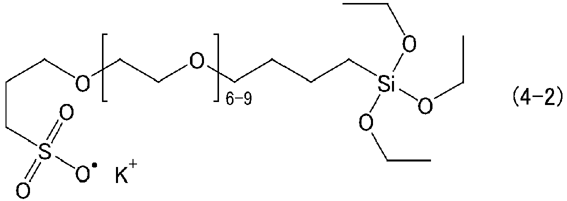

Examples of commercial products of the silane compound containing a metal salt-containing hydrophilic group include SIT8402.0 (a compound represented by formula (3-1)), SIT8378.5 (a compound represented by formula (4-1)), and SIT8192.2 (a compound represented by formula (4-2)), all available from Gelest.

The silane compound containing a halogen salt-containing hydrophilic group may be a silane compound containing a hydrolyzable group and a halogen salt-containing hydrophilic group in a molecule. Examples of the hydrolyzable group include a methoxy group and an ethoxy group. Examples of the halogen salt-containing hydrophilic group include ammonium halogen salts. The halogen of the halogen salt-containing hydrophilic group is not particularly limited. For improved water-holding properties, the halogen is preferably chlorine or bromine, more preferably chlorine.

The silane compound containing a halogen salt-containing hydrophilic group is preferably a compound containing an alkoxysilyl group that contains a hydrolyzable group, such as a methoxysilyl group (e.g. a trimethoxysilyl group) or an ethoxysilyl group (e.g. a triethoxysilyl group), or a compound containing a hydroxysilyl group (e.g. a trihydroxysilyl group), and particularly a compound containing an alkoxysilyl group.

In particular, the silane compound containing a halogen salt-containing hydrophilic group may suitably be, for example, a silane compound represented by the formula (5) below, or a silane compound containing a linkage unit 6A represented by the formula (6A) below and a linkage unit 6B represented by the formula (6B) below. From the standpoints of lubrication properties and sliding properties, it is preferably a silane compound containing a linkage unit 6A represented by formula (6A) and a linkage unit 6B represented by formula (6B), among these.

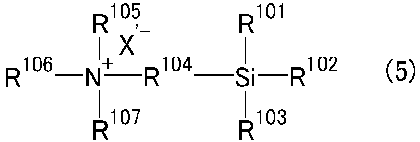

In the formula, R¹⁰¹, R¹⁰², and R¹⁰³ are the same as or different from each other and each represent a halogen atom, a hydroxy group, a linear or branched C1-C3 alkyl group, or a linear or branched C1-C3 alkoxy group, provided that at least one of R¹⁰¹, R¹⁰², and R¹⁰³ is a halogen atom, a hydroxy group, or a linear or branched C1-C3 alkoxy group; R¹⁰⁴ represents a linear or branched C1-C7 alkylene group; R¹⁰⁵, R¹⁰⁶, and R¹⁰⁷ are the same as or different from each other and each represent a hydrogen atom or a linear or branched C1-C5 alkyl group optionally containing an ether linkage; and X' represents a halogen atom.

From the standpoint of affinity for water, at least one of R¹⁰¹, R¹⁰², and R¹⁰³ is preferably a linear C1-C3 alkoxy group. From the same standpoint, R¹⁰⁴ is preferably a linear or branched C1-C5 alkylene group, more preferably a linear or branched C1-C4 alkylene group, still more preferably a linear or branched C1-C3 alkylene group.

From the standpoint of affinity for water, R¹⁰⁵, R¹⁰⁶, and R¹⁰⁷ are each preferably a hydrogen atom, a linear or branched C1-C5 alkyl group (e.g. a methyl group, an ethyl group, an isobutyl group), or a linear or branched C1-C5 alkoxyalkyl group (e.g. a methoxymethyl group, a methoxyethyl group, a methoxypropyl group, a methoxyisopropyl group). When the linear or branched C1-C5 alkyl group as R¹⁰⁵, R¹⁰⁶, or R¹⁰⁷ contains an ether linkage, the position of the ether linkage inserted is not particularly limited, and the number of ether linkages inserted per alkyl group may be one, or two or more.

From the standpoint of water-holding properties, X' is preferably a chlorine atom or a bromine atom, more preferably a chlorine atom.

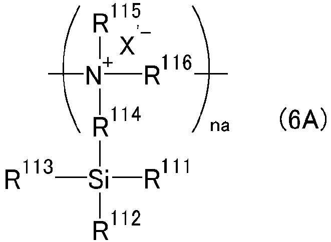

In the formula, R¹¹¹, R¹¹², and R¹¹³ are the same as or different from each other and each represent a halogen atom, a hydroxy group, a linear or branched C1-C3 alkyl group, or a linear or branched C1-C3 alkoxy group, provided that at least one of R¹¹¹, R¹¹², and R¹¹³ is a halogen atom, a hydroxy group, or a linear or branched C1-C3 alkoxy group; R¹¹⁴ represents a linear or branched C1-C7 alkylene group; R¹¹⁵ represents a hydrogen atom or a linear or branched C1-C5 alkyl group optionally containing an ether linkage; R¹¹⁶ represents a linear or branched C1-C5 alkylene group; X' represents a halogen atom; and na represents an integer of 1 or more.

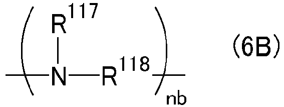

In the formula, R¹¹⁷ represents a hydrogen atom or a linear or branched C1-C5 alkyl group optionally containing an ether linkage; R¹¹⁸ represents a linear or branched C1-C5 alkylene group; and nb represents an integer of 1 or more.

Preferably, at least one of R¹¹¹, R¹¹², and R¹¹³ in formula (6A) is a linear C1-C3 alkoxy group. From the same standpoint as above, R¹¹⁴ is preferably a linear or branched C1-C5 alkylene group, more preferably a linear or branched C1-C4 alkylene group, still more preferably a linear or branched C1-C3 alkylene group.

From the standpoint of affinity for water, R¹¹⁵ in formula (6A) is preferably a hydrogen atom, a linear or branched C1-C5 alkyl group, or a linear or branched C1-C5 alkoxyalkyl group, more preferably a hydrogen atom. When the linear or branched C1-C5 alkyl group as R¹¹⁵ contains an ether linkage, the position of the ether linkage inserted is not particularly limited, and the number of ether linkages inserted per alkyl group may be one, or two or more.

From the standpoint of affinity for water, R¹¹⁶ in formula (6A) is preferably a linear or branched C1-C4 alkylene group, more preferably a linear or branched C1-C3 alkylene group. The symbol X' in formula (6A) is as described for X' in formula (5) . The symbol na is preferably 2 to 500, more preferably 3 to 100, still more preferably 3 to 50.

R¹¹⁷ in formula (6B) is as described for R¹¹⁵ in formula (6A). R¹¹⁸ is as described for R¹¹⁶ in formula (6A). The symbol nb is preferably 2 to 500, more preferably 3 to 100, still more preferably 3 to 50.

In the silane compound containing the linkage unit 6A and the linkage unit 6B, the sum (na+nb) of the number of repetitions of the linkage unit 6A (na) and the number of repetitions of the linkage unit 6B (nb) is preferably in the range of 2 to 1, 000, and more preferably in the range of 5 to 200.

In the silane compound containing the linkage unit 6A and the linkage unit 6B, the linkage unit 6A content is preferably 5 mol% or more, more preferably 10 mol% or more, but is preferably 75 mol% or less, more preferably 50 mol% or less, still more preferably 40 mol% or less. On the other hand, the linkage unit 6B content is preferably 25 mol% or more, more preferably 50 mol% or more, still more preferably 60 mol% or more, but is preferably 95 mol% or less, more preferably 90 mol% or less. In such cases, the effects of the present invention can be more suitably achieved.

The linkage unit 6A content and the linkage unit 6B content respectively include the linkage units 6A and 6B located at the ends of the silane compound. The forms of the linkage units 6A and 6B located at the ends of the silane compound are not particularly limited as long as they respectively correspond to the above formulas (6A) and (6B) representing the linkage units 6A and 6B, respectively.

Although the silane compound containing the linkage unit 6A and the linkage unit 6B may contain other linkage units, the combined content of the linkage units 6A and 6B is preferably 95 mol% or more, more preferably 98 mol% or more, particularly preferably 100 mol%. The order of the linkage units in the silane compound containing the linkage unit 6A and the linkage unit 6B is not particularly limited, and the linkage units may be arranged randomly, in blocks, or in an alternating fashion.

The silane compound containing the linkage unit 6A and the linkage unit 6B preferably has a weight average molecular weight in the range of 1,000 to 10,000. In this case, the effects of the present invention can be more suitably achieved.

In the present invention, the weight average molecular weight can be measured using a gel permeation chromatograph (GPC) calibrated with polystyrene standards.

Suitable examples of the silane compound represented by formula (5) and the silane compound containing a linkage unit 6A represented by formula (6A) and a linkage unit 6B represented by formula (6B) include compounds represented by the formulas (5-1) and (5-2) below, and compounds containing a linkage unit 6A-1 represented by the formula (6A-1) below and a linkage unit 6B-1 represented by the formula (6B-1) below.

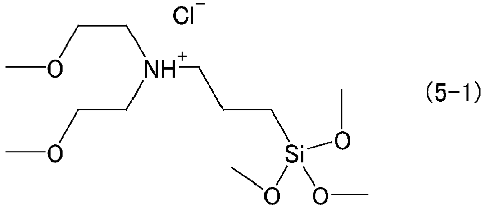

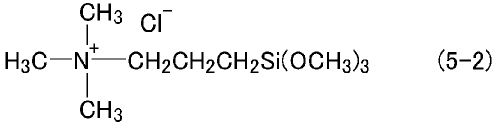

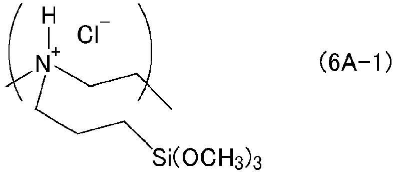

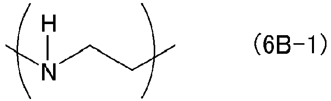

Examples of commercial products of the silane compound containing a halogen salt-containing hydrophilic group include SIB1500.0 (a compound represented by formula (5-1)), SIT8415.0 (a compound represented by formula (5-2)), and SSP-060 (a compound containing a linkage unit of formula (6A-1) and a linkage unit of formula (6B-1)), all available from Gelest.

Preferred examples of the silane compound containing a fluorine-containing hydrophobic group include silane compounds having a perfluoroether structure, specifically silane compounds containing a hydrolyzable group and a perfluoropolyether group in a molecule. Examples of the hydrolyzable group include a methoxy group and an ethoxy group. Examples of the perfluoropolyether group (perfluorooxyalkylene group) include divalent groups having a linear perfluoropolyalkylene ether structure. The silane compound containing a fluorine-containing hydrophobic group preferably contains a fluoroalkyl group such as a perfluoroalkyl group.

The silane compound containing a fluorine-containing hydrophobic group may suitably be a silane compound represented by the formula (7) or (8) below. 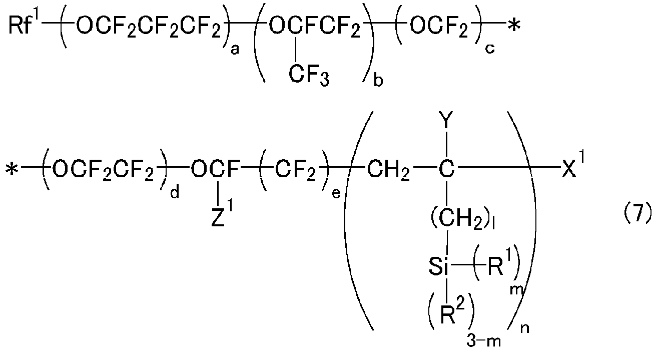 In the formula, Rf¹ represents a perfluoroalkyl group; Z¹ represents a fluorine atom or a trifluoromethyl group; a, b, c, d, and e are the same as or different from each other and each represent an integer of 0 or 1 or more, provided that a+b+c+d+e is 1 or more and the order of the repeating units parenthesized by subscripts a, b, c, d, and e occurring in the formula is not limited to that shown; Y represents a hydrogen atom or a C1-C4 alkyl group; X¹ represents a hydrogen atom, a bromine atom, or an iodine atom; R¹ represents a hydroxy group or a hydrolyzable substituent; R² represents a hydrogen atom or a monovalent hydrocarbon group; 1 represents 0, 1, or 2; m represents 1, 2, or 3; and n represents an integer of 1 or more, provided that the two ends marked by * are directly bonded to each other.

Rf¹ may be any perfluoroalkyl group that can be present in a common organic-containing fluoropolymer, including, for example, linear or branched C1-C16 perfluoroalkyl groups. Preferred are CF₃-, C₂F₅-, and C₃F₇-, among others.

Each of a, b, c, d, and e represents the number of repeating units in the perfluoropolyether chain which forms the backbone of the silane compound containing a fluorine-containing hydrophobic group, and is independently preferably 0 to 200, more preferably 0 to 50. Moreover, a+b+c+d+e (the sum of a to e) is preferably 1 to 100. The order of the repeating units parenthesized by subscripts a, b, c, d, and e occurring in formula (7) is not limited to the order shown, and the repeating units may be joined in any order.

Examples of the C1-C4 alkyl group represented by Y include a methyl group, an ethyl group, a propyl group, and a butyl group, and the group may be linear or branched. When X¹ is bromine or iodine, the silane compound containing a fluorine-containing hydrophobic group easily forms a chemical bond.

The hydrolyzable substituent represented by R¹ is not particularly limited. Preferred examples include halogens, -OR²⁰¹, -OCOR²⁰¹, -OC (R²⁰¹)=C (R²⁰²)₂, -ON=C(R²⁰¹)₂, and -ON=CR²⁰³, where R²⁰¹ represents an aliphatic hydrocarbon group or an aromatic hydrocarbon group; R²⁰² represents a hydrogen atom or a C1-C4 aliphatic hydrocarbon group; and R²⁰³ represents a C3-C6 divalent aliphatic hydrocarbon group. More preferred are chlorine, -OCH₃, and -OC₂H₅.

The monovalent hydrocarbon group represented by R² is not particularly limited, and preferred examples include a methyl group, an ethyl group, a propyl group, and a butyl group. The group may be linear or branched.

The symbol 1 represents the carbon number of the alkylene group between the carbon in the perfluoropolyether chain and the silicon attached thereto and is preferably 0. The symbol m represents the number of substituents R¹ bonded to the silicon to which R² is bonded through a bond not attached to R¹. The upper limit of n is not particularly limited, and is preferably an integer of 1 to 10.

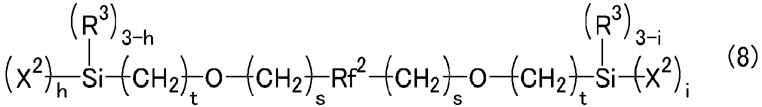

In the formula, Rf² represents a divalent group having a non-branched linear perfluoropolyalkylene ether structure that contains a unit represented by -(CₖF₂ₖ)O- where k is an integer of 1 to 6; each R³ is the same or different and represents a C1-C8 monovalent hydrocarbon group; each X² is the same or different and represents a hydrolyzable group or a halogen atom; each s is the same or different and represents an integer of 0 to 2; each t is the same or different and represents an integer of 1 to 5; and h and i are the same as or different from each other and each represent 2 or 3.

The Rf² group is preferably, but not limited to, such that when each s is 0, the ends of the Rf² group bonded to the oxygen atoms in formula (8) are not oxygen atoms. Moreover, k in Rf² is preferably an integer of 1 to 4. Specific examples of the Rf² group include -CF₂CF₂O(CF₂CF₂CF₂O)ⱼCF₂CF₂- in which j represents an integer of 1 or more, preferably of 1 to 50, more preferably of 10 to 40; and -CF₂(OC₂F₄)ₚ-(OCF₂)_{q}- in which p and q each represent an integer of 1 or more, preferably of 1 to 50, more preferably of 10 to 40, and the sum of p and q is an integer of 10 to 100, preferably of 20 to 90, more preferably of 40 to 80, and the repeating units (OC₂F₄) and (OCF₂) in the formula are randomly arranged.

R³ is preferably a C1-C3 monovalent hydrocarbon group, specifically, for example, a methyl group.

Examples of the hydrolyzable group represented by X² include alkoxy groups such as a methoxy group, an ethoxy group, a propoxy group, and a butoxy group; alkoxyalkoxy groups such as a methoxymethoxy group, a methoxyethoxy group, and an ethoxyethoxy group; alkenyloxy groups such as an allyloxy group and an isopropenoxy group; acyloxy groups such as an acetoxy group, a propionyloxy group, and a butylcarbonyloxy group; ketoxime groups such as a dimethylketoxime group and a methylethylketoxime group; amino groups such as an N-methylamino group, an N-ethylamino group, an N-propylamino group, an N-butylamino group, and an N,N-dimethylamino group; amide groups such as an N-methylacetamide group and an N-ethylacetamide group; and aminooxy groups such as an N,N-dimethylaminooxy group and an N,N-diethylaminooxy group. Examples of the halogen atom represented by X² include a chlorine atom, a bromine atom, and an iodine atom. Preferred among these are a methoxy group, an ethoxy group, an isopropenoxy group, and a chlorine atom.

The symbol s is preferably 1, while t is preferably 3. From the standpoint of hydrolyzability, each of h and i is preferably 3.

For durable mold-releasing effect, the silane compound containing a fluorine-containing hydrophobic group preferably has a weight average molecular weight in the range of 1,000 to 10,000.

Examples of commercial products of the silane compound containing a fluorine-containing hydrophobic group include OPTOOL DSX available from Daikin Industries, Ltd. and KY-130 available from Shin-Etsu Chemical Co., Ltd.

The production method further includes, after step 2, step 3 of maintaining the functionalized product (surface-treated metal medical device) obtained in step 2 at a humidity of 50% or more. This further improves the durability of the functional layer (lubrication layer), so that the effects of the present invention can be significantly achieved. The humidity is more preferably 60% or more, still more preferably 80% or more. The upper limit of the humidity is not particularly limited and is preferably 100%, for example.

The duration and temperature for maintaining the functionalized product at a humidity of 50% or more may be selected appropriately in view of the effects of the present invention. For example, the product is maintained preferably for 20 to 60 hours and preferably at 20°C to 60°C.

Sliding properties, low protein adsorption properties, low cell adsorption properties, and the durability of these properties can be suitably imparted to the metal medical device of the present invention produced, for example, as described above. The metal medical device may have the surface treatment layer including the primer layer and the functional layer on a part of its metal surface or on the entire metal surface.

The metal medical device may be used for guide wires, stents, needles, stylets, and metal tubes, for example. Particularly for guide wires, the effects of imparting lubrication properties, of improving the durability of the lubrication layer on the surface, and of reducing deterioration of sliding properties are available, and as a result insertion, pushing, and sliding properties in body cavities or tissues and the durability of these properties are improved, and thus the effects of the present invention can be significantly achieved. In the case of stents, the effects of imparting low protein adsorption properties or low cell adsorption properties, and of improving the durability of the lubrication layer on the surface (the effect of reducing deterioration of low protein adsorption properties and low cell adsorption properties) are available, and as a result the lubrication layer is prevented from flowing away during the long-term indwelling period and the effect of preventing adhesion of proteins and cells to the surface of the stent is maintained, and thus the effects of the present invention can be significantly achieved.

Examples of the material of the metal medical device include metals such as stainless steel, nickel-titanium alloys, iron, titanium, aluminum, tin, and zinc-tungsten alloys. Stainless steel or nickel-titanium alloys are especially suitable.

### EXAMPLES

The present invention will be specifically described with reference to examples below, but is not limited to the examples.

### (Example 1)

A SUS guide wire (core wire) was washed with acetone and dried. The dried guide wire was immersed in Primer coat PC-3B (available from Fluoro Technology, a butoxy/ethoxy type tetraalkoxysilane represented by formula (II-1)), pulled out, and dried.

The dried primed guide wire was immersed in a 10% by mass aqueous solution of SIM6492.57 (available from Gelest, a compound represented by formula (1-3)) for 30 minutes, taken out, and allowed to stand at room temperature (25°C) and a humidity of 90% for 24 hours. Thereafter, the guide wire was washed with water and dried to obtain a surface-treated guide wire.

### (Example 2)

A surface-treated guide wire was prepared as in Example 1, except that an aqueous solution of SIB1824.84 (available from Gelest, a compound represented by formula (2-1)) was used instead of the SIM6492.57 aqueous solution.

### (Example 3)

A surface-treated guide wire was prepared as in Example 1, except that an aqueous solution of SIT8402.0 (available from Gelest, a compound represented by formula (3-1)) was used instead of the SIM6492.57 aqueous solution.

### (Example 4)

A surface-treated guide wire was prepared as in Example 1, except that an aqueous solution of SIT8378.5 (available from Gelest, a compound represented by formula (4-1)) was used instead of the SIM6492.57 aqueous solution.

### (Example 5)

A surface-treated guide wire was prepared as in Example 1, except that a 0.1% by mass solution of OPTOOL DSX (available from Daikin Industries, Ltd., a silane compound containing a fluorine-containing hydrophobic group) in perfluorohexane was used instead of the SIM6492.57 aqueous solution.

### (Example 6)

A surface-treated guide wire was prepared as in Example 1, except that a nickel-titanium alloy guide wire (core wire) was used instead of the SUS guide wire (core wire).

### (Example 7)

A surface-treated guide wire was prepared as in Example 2, except that a nickel-titanium alloy guide wire (core wire) was used instead of the SUS guide wire (core wire).

### (Example 8)

A surface-treated guide wire was prepared as in Example 3, except that a nickel-titanium alloy guide wire (core wire) was used instead of the SUS guide wire (core wire).

### (Example 9)

A surface-treated guide wire was prepared as in Example 4, except that a nickel-titanium alloy guide wire (core wire) was used instead of the SUS guide wire (core wire).

### (Example 10)

A surface-treated guide wire was prepared as in Example 5, except that a nickel-titanium alloy guide wire (core wire) was used instead of the SUS guide wire (core wire).

### (Example 11)

A surface-treated guide wire was prepared as in Example 1, except that an aqueous solution of SIT8192.2 (available from Gelest, a compound represented by formula (4-2)) was used instead of the SIM6492.57 aqueous solution.

### (Example 12)

A surface-treated guide wire was prepared as in Example 1, except that an aqueous solution of SIB1500.0 (available from Gelest, a compound represented by formula (5-1)) was used instead of the SIM6492.57 aqueous solution.

### (Example 13)

A surface-treated guide wire was prepared as in Example 1, except that an aqueous solution of SIB1500.0 (available from Gelest, a compound represented by formula (5-1)) whose pH was adjusted to 4 with acetic acid was used instead of the SIM6492.57 aqueous solution.

### (Example 14)

A surface-treated guide wire was prepared as in Example 1, except that an aqueous solution of SSP-060 (available from Gelest, a compound containing a linkage unit of formula (6A-1) and a linkage unit of formula (6B-1)) was used instead of the SIM6492.57 aqueous solution.

### (Comparative Example 1)

A SUS guide wire (core wire) only washed with acetone and dried was used.

### (Comparative Example 2)

A nickel-titanium alloy guide wire (core wire) only washed with acetone and dried was used.

The surface-treated guide wires or guide wires obtained in the examples and comparative examples were evaluated as follows.

### <Evaluation of sliding properties>

After water was put on each surface-treated guide wire or guide wire, the guide wire was rubbed by a hand to subjectively evaluate sliding properties.

### (Evaluation results)

The evaluation on the SUS guide wires revealed that the surface-treated guide wires of Examples 1 to 5 and 11 to 14 had a slippery surface and exhibited good sliding properties as compared to the guide wire of Comparative Example 1. Particularly, the guide wires of Examples 11 and 14 had a highly slippery surface. Moreover, they also showed no change in slipperiness after rubbing 100 times, indicating excellent ability to reduce deterioration of sliding properties. Similarly, the evaluation on the nickel-titanium alloy guide wires revealed that the surface-treated guide wires of Examples 6 to 10 exhibited good sliding properties and excellent ability to reduce deterioration of sliding properties as compared to the guide wire of Comparative Example 2.

### <Protein adsorption properties and cell adsorption properties>

The adsorption of proteins and cells onto the surface-treated guide wires and guide wires was evaluated.

### (Evaluation results)

The surface-treated guide wires of Examples 1, 2, 6, and 7 in which a functional layer was formed of a silane compound containing a polyoxyalkylene group on a primer layer showed low adsorption of proteins and cells. The surface-treated guide wires of Examples 3, 4, 8, 9, and 11 using a silane compound containing a metal salt-containing hydrophilic group showed lower adsorption. The surface-treated guide wires of Examples 12 to 14 using a silane compound containing a halogen salt-containing hydrophilic group showed much lower adsorption than the untreated guide wires (metals) of Comparative Examples 1 and 2, though their adsorption amounts were slightly higher than those obtained using a silane compound containing a metal salt-containing hydrophilic group. The surface-treated guide wires of Examples 5 and 10 using a silane compound containing a fluorine-containing hydrophobic group also showed low adsorption. Moreover, the surface-treated guide wires of Examples maintained low adsorption properties after repeating adsorption of proteins and cells and washing.

The above results demonstrated that metal medical devices having on its metal surface a surface treatment layer including a certain primer layer and a certain silane compound layer are excellent in sliding properties, low protein adsorption properties, low cell adsorption properties, and the durability of these properties (the ability to reduce deterioration of sliding properties, low protein adsorption properties, and low cell adsorption properties).

## Claims

1. A method for producing a silane-functionalized metal medical device comprising a surface treatment layer on at least a part of its metal surface, the surface treatment layer comprising: a primer layer formed of an alkoxysilane; and a functional layer on the primer layer, the functional layer being formed of a silane compound containing a polyoxyalkylene group, a metal salt-containing hydrophilic group, a halogen salt-containing hydrophilic group, or a fluorine-containing hydrophobic group, the method comprising:
step 1 of priming a metal surface with an alkoxysilane; and
step 2 of functionalizing the primed surface using a silane compound containing a polyoxyalkylene group, a metal salt-containing hydrophilic group, a halogen salt-containing hydrophilic group, or a fluorine-containing hydrophobic group,
wherein the method further comprises step 3 of maintaining a functionalized product obtained in step 2 at a humidity of 50% or more,
wherein the alkoxysilane is represented by the formula (I):
R¹¹ₖSi(OR¹²)₄₋ₖ (I),
wherein each R¹¹ or each R¹² is the same or different and represents a C1-C8 saturated or unsaturated hydrocarbon group, and R¹¹ and R¹² are the same as or different from each other, and k represents an integer of 0 to 3.

2. The method according to claim 1,
wherein step 2 is carried out at a pH of 5 or lower.

## Patentansprüche

1. Verfahren zur Herstellung einer silanfunktionalisierten medizinischen Metallvorrichtung, die auf zumindest einem Teil ihrer Metalloberfläche eine Oberflächenbehandlungsschicht umfasst, wobei die Oberflächenbehandlungsschicht umfasst: eine mit einem Alkoxysilan gebildete Grundierungsschicht; und eine Funktionsschicht auf der Grundierungsschicht, wobei die Funktionsschicht mit einer Silanverbindung gebildet ist, die eine Polyoxyalkylengruppe, eine metallsalz-haltige hydrophile Gruppe oder eine fluorhaltige hydrophobe Gruppe enthält, wobei das Verfahren umfasst:
Schritt 1 des Grundierens einer Metalloberfläche mit einem Alkoxysilan; und
Schritt 2 des Funktionalisierens der grundierten Oberfläche unter Verwendung einer Silanverbindung, die eine Polyoxyalkylengruppe, eine metallsalz-haltige hydrophile Gruppe oder eine fluorhaltige hydrophobe Gruppe enthält,
wobei das Verfahren zudem Schritt 3 des Haltens eines in Schritt 2 erhaltenen funktionalisierten Produkts bei einer Luftfeuchtigkeit von 50% oder mehr umfasst,
wobei das Alkoxysilan durch die Formel (I) dargestellt ist:
R¹¹ₖSi(OR¹²)₄₋ₖ (I),
wobei jedes R¹¹ oder jedes R¹² gleich oder verschieden ist und eine gesättigte oder ungesättigte C1-C8-Kohlenwasserstoffgruppe darstellt, und R¹¹ und R¹² gleich oder voneinander verschieden sind, und k eine ganze Zahl von 0 bis 3 darstellt.

2. Verfahren nach Anspruch 1,
wobei Schritt 2 bei einem pH von 5 oder niedriger durchgeführt wird.

## Revendications

1. Procédé de production d'un dispositif médical en métal avec une fonction silane comprenant une couche de traitement de surface sur au moins une partie de sa surface métallique, la couche de traitement de surface comprenant : une couche de primaire formée d'un alcoxysilane ;
et une couche fonctionnelle sur la couche de primaire, la couche fonctionnelle étant formée d'un composé silane contenant un groupe polyoxyalkylène, un groupe hydrophile contenant un sel métallique, un groupe hydrophile contenant un sel halogéné, ou un groupe hydrophile contenant du fluor, le procédé comprenant :
une étape 1 de revêtement par un primaire d'une surface métallique avec un alcoxysilane ;
et
une étape 2 de fonctionnalisation de la surface revêtue de primaire en utilisant un composé silane contenant un groupe polyoxyalkylène, un groupe hydrophile contenant un sel métallique, un groupe hydrophile contenant un sel halogéné, ou un groupe hydrophile contenant du fluor, ledit procédé comprenant en outre une étape 3 de maintien d'un produit fonctionnalisé obtenu dans l'étape 2 à une humidité de 50 % ou plus,
dans lequel l'alcoxysilane est représenté par la formule (I):
R¹¹ₖSi(OR¹²)₄₋ₖ (I),
dans lequel chaque R¹¹, ou chaque R¹², est identique ou différent, et représente un groupe hydrocarboné en C1 à C8, saturé ou insaturé, et R¹¹ et R¹² sont identiques ou différents l'un de l'autre, et k représente un nombre entier de 0 à 3.

2. Procédé selon la revendication 1,
dans lequel l'étape 2 est effectuée à un pH de 5 ou moins.
